# FASCICULE DE BREVET EUROPEEN

(11) **EP 0 966 948 B1**
(45) Date de publication et mention de la délivrance du brevet: **22.05.2002**
(21) Numéro de dépôt: 99401432.2
(22) Date de dépôt: 11.06.1999
(51) Int. Cl.: A61K 7/06, A61K 7/48

(54) **Composition cosmétique contenant un polymère anionique et un terpolymère acrylique et utilisation de cette composition pour le traitement des matières kératiniques**
Kosmetische Zusammensetzung enthaltend ein anionisches Polymer und ein acrylisches Terpolymer und Verwendung dieser Zusammensetzung zur Behandlung von Keratinfasern
Cosmetic composition containing an anionic polymer and an acrylic terpolymer and use of said composition for treating keratinic materials

(30) Priorité: 15.06.1998 FR 9807514
(43) Date de publication de la demande: 29.12.1999
(73) Titulaire: L'OREAL, 75008 Paris (FR)
(72) Inventeur: Dupuis, Christine, 75018 Paris (FR)
(74) Mandataire: Casalonga, Axel

(56) Documents cités:
- WO-A-93/24544

## Description

La présente invention concerne des compositions cosmétiques contenant en association, au moins un polymère anionique et un terpolymère acrylique, ainsi que l'utilisation de ces compositions pour le traitement des matières kératiniques, en particulier la peau et les cheveux.

Les polymères anioniques sont généralement utilisés pour leurs propriétés fixantes, notamment pour la formulation des gels capillaires de coiffage et de fixation.

Il est intéressant de formuler, pour le soin et le coiffage, des compositions capillaires contenant des polymères anioniques qui ont une viscosité élevée et se présentent sous forme de gels épais s'étalant bien,.

Pour cela, on fait en général appel à des polymères épaississants et/ou gélifiants. Cependant, l'introduction des polymères anioniques dans les épaississants conduit souvent à des problèmes de fluidification et de perte de limpidité du support et les performances cosmétiques obtenues sont parfois insuffisantes pour des produits de soin et de coiffage.

On connaît des polymères épaississants et/ou gélifiants comportant dans leur chaîne une partie hydrophile et une partie hydrophobe constituée d'une chaîne grasse comme le produit "PEMULEN TR1" commercialisé par la société GOODRICH ou les polymères "ACRYSOL" commercialisés par la société ROHM & HAAS. Le polymère "PEMULEN TR1", utilisé en association avec des polymères anioniques, ne conduit pas à un gel de texture et de transparence satisfaisantes et ne donne pas sur le plan cosmétique des résultats satisfaisants, notamment en ce qui concerne le pouvoir fixant, le démêlage, la douceur et le toucher. Le polymère "ACRYSOL 44", utilisé en association avec un polymère anionique, conduit à un gel fluide et trouble.

On connaît aussi par la demande de brevet WO-A-93 24544 des polymères épaississants utilisés dans des compositions aqueuses de revêtement et plus spécialement dans les peintures latex, obtenus par réaction:
(A) d'environ 1-99,9% en poids d'un ou plusieurs acides carboxyliques à insaturation α,β-monoéthylénique, typiquement l'acide méthacrylique;
(B) d'environ 0-98,9% en poids d'un ou plusieurs monomères à insaturation monoéthylénique différents de (A), typiquement l'acrylate d'éthyle;
(C) d'environ 0,1-99% en poids d'un ou plusieurs macromonomères à insaturation monoéthylénique différents de (A) et (B);
(D) d'environ 0-20% en poids ou plus d'un ou plusieurs monomères à insaturation polyéthylénique différents de (A), (B) et (C).

La demanderesse a découvert de manière surprenante qu'en utilisant une nouvelle famille de polymères épaississants et/ou gélifiants et en les associant à des polymères anioniques, on pouvait obtenir des formulations cosmétiques présentant une viscosité satisfaisante à un pH relativement peu élevé, non pâteuses, non grasses, qui s'étalent bien sur la peau et les cheveux et qui confèrent à ces derniers de bonnes propriétés de douceur, toucher et démêlage tout en ayant de bonnes propriétés coiffantes et/ou fixantes.

La présente invention a donc pour objet des compositions cosmétiques pour le traitement des matières kératiniques contenant, dans un support aqueux cosmétiquement acceptable, au moins un polymère anionique et un terpolymère acrylique que l'on définira plus en détail dans la suite de la description.

Ce polymère permet notamment de préparer des compositions aqueuses ou hydro-organiques contenant des solvants cosmétiquement acceptables, rincées ou non rincées, allant des produits gélifiés aux sticks ou bâtonnets solides.

Les avantages de ce terpolymère sont d'être stable en milieu électrolyte et d'avoir un très bon pouvoir épaississant à pH égal ou supérieur à 5,5 permettant d'atteindre un bon niveau de viscosité et de pouvoir utiliser des concentrations élevées en alcool.

Le terpolymère acrylique utilisé conformément à l'invention est soluble ou gonflable dans les alcalis. Il est caractérisé par le fait qu'il comprend :
a) 20 à 70% en poids, de préférence 25 à 55% en poids, d'un acide carboxylique à insaturation α, β-monoéthylénique ;
b) 20 à 65% en poids, de préférence 30 à 65% en poids, d'un monomère à insaturation monoéthylénique non tensio-actif différent de a) et
c) 0,5 à 60% en poids, de préférence 10 à 50% en poids, d'un monomère uréthane non-ionique qui est le produit de réaction d'un tensio-actif non-ionique monohydrique avec un monoisocyanate à insaturation monoéthylénique.

L'acide carboxylique à insaturation α, β-monoéthylénique a) peut être choisi parmi de nombreux acides et en particulier l'acide acrylique, l'acide méthacrylique, l'acide itaconique et l'acide maléique. L'acide méthacrylique est préféré. Une large proportion d'acide est essentielle pour donner une structure polymère qui se solubilise et donne un épaississant par réaction avec un composé alcalin comme l'hydroxyde de sodium, les alcanolamines, l'amino méthyl propanol ou l'amino méthyl propanediol.

Le terpolymère doit aussi contenir une proportion importante indiquée ci-dessus d'un monomère b) à insaturation monoéthylénique qui n'a pas de propriété tensio-active. Les monomères préférés sont ceux qui donnent des polymères insolubles dans l'eau lorsqu'ils sont homopolymérisés et sont illustrés par les acrylates et méthacrylates d'alkyle en C₁-C₄ comme l'acrylate de méthyle, l'acrylate d'éthyle, l'acrylate de butyle ou les méthacrylates correspondants. Les monomères plus particulièrement préférés sont les acrylates de méthyle et d'éthyle. D'autres monomères pouvant être utilisés sont le styrène, le vinyltoluène, l'acétate de vinyle, l'acrylonitrile et le chlorure de vinylidène. Les monomères non réactifs sont préférés, ces monomères étant ceux dans lesquels le groupe éthylénique unique est le seul groupe réactif dans les conditions de la polymérisation. Cependant, des monomères qui contiennent des groupes réactifs sous l'action de la chaleur peuvent être utilisés dans certaines situations, comme l'acrylate d'hydroxyéthyle.

Les tensio-actifs non-ioniques monohydriques utilisés pour obtenir le monomère uréthane non-ionique c) sont bien connus et sont généralement des composés hydrophobes alcoxylés contenant un oxyde d'alkylène formant la partie hydrophile de la molécule. Les hydrophobes sont généralement constitués par un alcool aliphatique ou un alkylphénol dans lesquels une chaîne carbonée contenant au moins six atomes de carbone constitue la partie hydrophobe du tensio-actif.

Les tensio-actifs non ioniques monohydriques préférés ont pour formule : 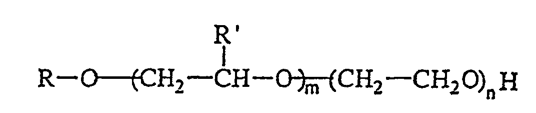 dans laquelle R est un groupe alkyle en C₆-C₃₀ ou aralkyle en C₈-C₃₀, R' est un groupe alkyle en C₁-C₄, n est un nombre moyen allant de 5 à 150 et m est un nombre moyen allant de 0 à 50, à la condition que n soit au moins aussi grand que m et que n + m = 5-150.

A titre de groupes alkyle en C₆-C₃₀ préférés, on peut citer les radicaux dodécyle et alkyle en C₁₈-C₂₆. A titre de groupes aralkyle, on peut citer plus particulièrement les groupes alkyl (C₈-C₁₃) phényle. Le groupe R' préféré est le groupe méthyle.

Le monoisocyanate à insaturation monoéthylénique utilisé pour former le monomère uréthane non-ionique c) peut être choisi parmi des composés très variés. On peut utiliser un composé contenant toute insaturation copolymérisable telle qu'une insaturation acrylique ou méthacrylique. On peut aussi utiliser une insaturation allylique conférée par l'alcool allylique. Le monoisocyanate monoéthylénique préféré est l'α,α-diméthyl-m-isopropényl-benzylisocyanate.

Le terpolymère acrylique défini ci-dessus est obtenu par copolymérisation en émulsion aqueuse des composants a), b) et c) qui est tout à fait usuelle et décrite dans la demande de brevet EP-A-0 173 109.

A titre de terpolymères pouvant être utilisés selon l'invention, on peut citer les produits de réaction de l'acide méthacrylique comme composant a), de l'acrylate d'éthyle comme composant b) et d'un macromonomère uréthane non-ionique comme composant c), ayant la structure suivante : 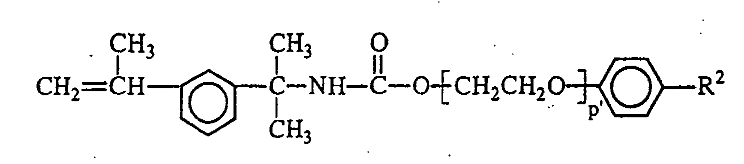 dans laquelle p' va de 6 à 150 et est égal de préférence à 30 et R² est un radical alkyle en C₈-C₁₃, tels que celui décrit dans l'exemple 3 de la demande de brevet EP-A-0 173 109.

Le terpolymère acrylique préféré utilisé selon l'invention est obtenu à partir d'acide méthacrylique comme composant a), d'acrylate de méthyle comme composant b) et d'un macromonomère uréthane non-ionique comme composant (c), ayant la structure suivante : 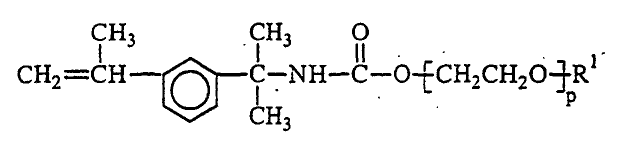 dans laquelle p va de 6 à 150 et R¹ est un radical alkyle en C₁₈-C₂₆, de préférence en C₂₀-C₂₄ linéaire, d'origine végétale tel que le radical docosyle.

Le terpolymère acrylique est présent dans les compositions cosmétiques de l'invention dans des concentrations allant de 0,01 à 20% en poids par rapport au poids total de la composition et préférentiellement de 0,1 à 10% en poids.

Selon l'invention, on peut utiliser tout polymère anionique connu en soi. Bien entendu, on peut utiliser un ou plusieurs polymères anioniques.

Ainsi, les polymères anioniques généralement utilisés sont des polymères comportant des groupements dérivés d'acide carboxylique, sulfonique ou phosphorique et ont un poids moléculaire compris entre environ 500 et 5.000.000.

Les groupements carboxyliques sont apportés par des monomères mono- ou diacides carboxyliques insaturés tels que ceux répondant à la formule: 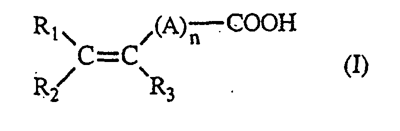 dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₁ désigne un atome d'hydrogène, un groupement phényle ou benzyle, R₂ désigne un atome d'hydrogène, un groupement alkyle inférieur ou carboxyle, R₃ désigne un atome d'hydrogène, un groupement alkyle inférieur, un groupement -CH₂ COOH, phényle ou benzyle.

Dans la formule précitée un radical alkyle inférieur désigne de préférence un groupement ayant 1 à 4 atomes de carbone et en particulier, méthyle et éthyle.

Les polymères anioniques à groupements carboxyliques préférés selon l'invention sont:
**A**) les homo- ou copolymères d'acide acrylique ou méthacrylique ou leurs sels et en particulier les produits vendus sous les dénominations "VERSICOL E" ou "VERSICOL K" par la société ALLIED COLLOID et "ULTRAHOLD" par la société BASF; les copolymères d'acide acrylique et d'acrylamide vendus sous la forme de leur sel de sodium sous les dénominations "RETEN 421", "RETEN 423" ou "RETEN 425" par la Société HERCULES ; les sels de sodium des acides polyhydroxycarboxyliques.
**B**) Les copolymères des acides acrylique ou méthacrylique avec au moins un monomère monoéthylénique tel que l'éthylène, le styrène, les esters vinyliques, les esters d'acide acrylique ou méthacrylique, l'acrylamide ou ses dérivés et la vinylpyrrolidone; éventuellement greffés sur un polyalkylène glycol tel que le polyéthylène glycol et éventuellement réticulés. De tels polymères sont décrits en particulier dans le brevet français 1.222.944 et la demande allemande 2.330.956. Les copolymères de ce type comportant dans leur chaîne un motif acrylamide éventuellement N-alkylé et/ou hydroxyalkylé sont décrits notamment dans les brevets FR 2 360 615 et FR 2 432 528 ou proposés sous la dénomination "QUADRAMER" par la Société AMERICAN CYANAMID. On peut également citer les copolymères d'acide acrylique et de méthacrylate d'alkyle en C₁-C₄ et les terpolymères de vinylpyrrolidone, d'acide (méth)acrylique et de (méth)acrylate d'alkyle en C₁-C₂₀, par exemple de lauryle tel que celui vendu par la société ISP sous la dénomination "ACRYLIDONE LM", de tertiobutyle, par exemple "LUVIFLEX VBM 70" commercialisé par BASF ou de méthyle, tel que "STEPANHOLD EXTRA" commercialisé par STEPAN. On peut également citer les copolymères d'acide méthacrylique et d'acrylate d'éthyle tels que celui vendu sous la dénomination "LUVIMER MAEX" par la société BASF, et les terpolymères acide méthacrylique/acrylate d'éthyle/acrylate de tertiobutyle tels que le produit "LUVIMER 100P" commercialisé par BASF.
**C**) Les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés sur un polyalkylèneglycol et réticulés ou encore un ester vinylique, allylique ou méthallylique d'un acide carboxylique α- ou β-cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 vendues par la société NATIONAL STARCH. On peut également citer le terpolymère acétate de vinyle/p-tert.butyl benzoate de vinyle/acide crotonique (65/25/10).
**D**) Les copolymères dérivés d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US 2.047.398, 2.723.248, 2.102.113, le brevet GB 839.805 et sont vendus notamment sous les dénominations "GANTREZ AN" ou "GANTREZ ES" ou "AVANTAGE CP" par la société ISP.
   - les copolymères comprenant (i) un ou plusieurs anhydrides maléique, citraconique ou itaconique et (ii) au moins un monomère choisi parmi les esters allyliques ou méthallyliques comportant éventuellement dans leur chaîne un ou plusieurs groupements acrylamide, méthacrylamide, α-oléfine, esters acryliques ou méthacryliques, acides acrylique ou méthacrylique ou vinylpyrrolidone, les fonctions anhydrides de ces copolymères étant éventuellement monoestérifiées ou monoamidifiées. Ces polymères sont par exemple dans les brevets français 2.350.384 et 2.357.241 de la demanderesse.
**E**) Les polyacrylamides comportant des groupements carboxylates.

Les polymères comprenant des groupements sulfoniques sont des polymères comportant des motifs vinylsulfonique, styrène sulfonique, naphtalène sulfonique ou acrylamido alkylsulfonique.

Ces polymères peuvent être notamment choisis parmi:
- les sels de l'acide polyvinylsulfonique ayant un poids moléculaire moyen en poids compris entre environ 1.000 et 100.000 ainsi que leurs copolymères avec un comonomère insaturé tel que les acides acrylique ou méthacrylique et leurs esters, l'acrylamide ou ses dérivés, les éthers vinyliques et la vinylpyrrolidone.
- les sels de l'acide polystyrène sulfonique, les sels de sodium ayant un poids moléculaire moyen en poids d'environ 500.000 et d'environ 100.000 étant vendus respectivement sous les dénominations "FLEXAN 500" et "FLEXAN 130" par NATIONAL STARCH. Ces composés sont décrits dans le brevet FR 2.198.719.
- les sels d'acides polyacrylamide alkylsulfoniques tels que ceux mentionnés dans le brevet US 4.128.631 et plus particulièrement l'acide polyacrylamidoéthylpropane sulfonique vendu sous la dénomination "COSMEDIA POLYMER HSP 1180" par HENKEL et l'acide polyacrylamidométhylpropane sulfonique réticulé et partiellement neutralisé à 50% par l'ammoniaque vendu sous la dénomination "HOSTACERIN AMPS" par la société HOECHST.

Selon l'invention, les polymères anioniques sont de préférence choisis parmi les copolymères d'acide acrylique ou méthacrylique avec au moins un monomère monoéthylénique, éventuellement greffés sur un polyalkylèneglycol et éventuellement réticulés tels que le terpolymère acide acrylique/acrylate d'éthyle/N-tertiobutylacrylamide vendu sous la dénomination "ULTRAHOLD STRONG" par la société BASF, les copolymères d'acide méthacrylique et de méthacrylate de méthyle vendus sous la dénomination "EUDRAGIT L" par la société ROHM PHARMA, le copolymère d'acide méthacrylique et d'acrylate d'éthyle vendu sous la dénomination "LUVIMER MAEX" par la société BASF, le terpolymère vinylpyrrolidone/acide acrylique/méthacrylate de lauryle vendu sous la dénomination "ACRYLIDONE LM" par la société ISP, les copolymères dérivés d'acide crotonique tels que le terpolymère acétate de vinyle/tertio-butyl benzoate de vinyle/acide crotonique, le terpolymère acide crotonique/acétate de vinyle/néododécanoate de vinyle vendu sous la dénomination "Résine 28-29-30" par la société NATIONAL STARCH, le copolymère acétate de vinyle/acide crotonique vendu sous la dénomination "LUVISET CA 66" par la société BASF et le terpolymère acétate de vinyle/acide crotonique/polyéthylèneglycol sous la dénomination "ARISTOFLEX A" par la société BASF, les copolymères dérivés d'acides ou d'anhydrides maléique, fumarique, itaconique tels que le copolymère méthylvinyléther/anhydride maléique monoestérifié vendu sous la dénomination "GANTREZ ES 425" par la société ISP.

Les polymères anioniques les plus particulièrement. préférés sont choisis parmi le terpolymère acétate de vinyle/tertio-butyl benzoate de vinyle/acide crotonique, le copolymère méthylvinyléther/anhydride maléique monoestérifié vendu sous la dénomination "GANTREZ ES 425" par la société ISP, le copolymère d'acide méthacrylique et de méthacrylate de méthyle vendu sous la dénomination "EUDRAGIT L" par la société ROHM PHARMA, le copolymère d'acide méthacrylique et d'acrylate d'éthyle vendu sous la dénomination "LUVIMER MAEX" par la société BASF, le terpolymère vinylpyrrolidone/acide acrylique/méthacrylate de lauryle vendu sous la dénomination "ACRYLIDONE LM" par la société ISP.

Selon l'invention, on peut également utiliser des polymères anioniques sous forme de latex ou de pseudolatex, c'est-à-dire sous forme d'une dispersion aqueuse de particules insolubles de polymères.

On peut par exemple utiliser une dispersion aqueuse comprenant un copolymère formé d'un ou plusieurs acrylates d'alkyle, d'un ou plusieurs méthacrylates d'alkyle et d'un ou plusieurs acides carboxyliques éthyléniques ayant de 3 à 5 atomes de carbone, les radicaux alkyle ayant de 1 à 5 atomes de carbone et pouvant être hydroxylés.

L'acrylate d'alkyle est de préférence choisi parmi l'acrylate de méthyle, l'acrylate d'éthyle, l'acrylate de propyle et l'acrylate de butyle. L'acrylate d'éthyle est particulièrement préféré.

La concentration, en acrylate d'alkyle est de préférence comprise entre 40 et 70% en poids et plus particulièrement entre 50 et 60% en poids par rapport au poids total du copolymère.

Le méthacrylate d'alkyle est de préférence choisi parmi le méthacrylate de méthyle, le méthacrylate d'éthyle, le méthacrylate de propyle et le méthacrylate de butyle. Le méthacrylate de méthyle est particulièrement préféré.

La concentration en méthacrylate d'alkyle est de préférence comprise entre 30 et 50% en poids et plus particulièrement entre 30 et 40% en poids par rapport au poids total du copolymère.

Les acides carboxyliques éthyléniques préférés sont l'acide acrylique, l'acide méthacrylique, l'acide crotonique, l'acide itaconique ou leurs mélanges. L'acide acrylique et l'acide méthacrylique sont particulièrement préférés. Selon l'invention, il est possible de mettre en oeuvre des sels de ces acides carboxyliques.

La concentration en acides carboxyliques éthyléniques, ou leurs sels, est de préférence comprise entre 5 et 15% en poids et plus particulièrement entre 8 et 12% en poids par rapport au poids total du copolymère.

Dans un mode de réalisation particulièrement préféré de l'invention, l'acide acrylique est utilisé avec l'acide méthacrylique, chacun dans une concentration comprise entre 2 et 10% en poids, le total de ces deux acides n'excédant pas 15% en poids du poids total du copolymère.

Le copolymère peut également contenir des faibles quantités, c'est à dire moins de 10%, de préférence moins de 5% et plus particulièrement moins de 2%, d'un monomère polymérisable autre que ceux mentionnés ci-dessus.

Selon un mode particulièrement préféré de mise en oeuvre de l'invention, on utilise un copolymère comprenant de 50 à 60% en poids d'acrylate d'éthyle, de 30 à 40% en poids de méthacrylate de méthyle. de 2 à 10% en poids d'acide acrylique, de 2 à 10% en poids d'acide méthacrylique, la concentration totale d'acide acrylique et méthacrylique n'excédant pas 15% en poids par rapport au poids total du copolymère acrylique.

Un tel copolymère est par exemple décrit dans la demande de brevet EP-A-590604.

Une dispersion aqueuse du copolymère acrylique décrit ci-dessus comprenant 25% en poids d'un copolymère acrylate d'éthyle/méthacrylate de méthyle/acide méthacrylique/ acide acrylique est vendue notamment sous la dénomination commerciale "AMERHOLD DR-25" par la société AMERCHOL.

Selon l'invention, on peut également utiliser une dispersion aqueuse de copolymère méthacrylate d'hydroxyéthyle/méthacrylate de méthyle/acide méthacrylique/acrylate de butyle telle que par exemple le produit commercialisé par la société SEPPIC sous la dénomination "ACUDYNE255".

Selon l'invention, on peut également utiliser une dispersion aqueuse de copolymère acrylate d'éthyle/acide méthacrylique/acrylate de tert.butyle telle que par exemple le produit commercialisé par la société BASF sous la dénomination "LUVIMER LOW VOC" .

Les polymères anioniques sont utilisés dans les compositions de l'invention dans des proportions comprises entre 0,01 et 20% en poids et de préférence entre 0,1 et 8% en poids par rapport au poids total de la composition.

Les compositions selon l'invention contiennent un milieu aqueux cosmétiquement acceptable. Elles présentent un pH pouvant aller de 3,5 à 11, de préférence de 5,5 à 11 et encore plus préférentiellement de 5,5 à 8,5.

Le milieu cosmétiquement acceptable des compositions selon l'invention est plus particulièrement constitué d'eau et éventuellement de solvants organiques cosmétiquement acceptables.

Les solvants organiques peuvent représenter de 0,5 à 90% du poids total de la composition. Ils peuvent être choisis dans le groupe constitué par les solvants organiques hydrophiles, les solvants organiques lipophiles, les solvants amphiphiles ou leurs mélanges.

Parmi les solvants organiques hydrophiles, on peut citer par exemple les mono-alcools inférieurs linéaires ou ramifiés ayant de 1 à 8 atomes de carbone, les polyéthylène glycols ayant de 6 à 80 unités d'oxyde d'éthylène et les polyols.

Comme solvants organiques amphiphiles, on peut citer les dérivés de polypropylène glycol (PPG) comme les esters de polypropylène glycol et d'acide gras, de PPG et d'alcool gras comme le PPG-23 oléyl éther et le PPG-36 oléate.

Comme solvants organiques lipophiles, on peut citer par exemple les esters gras tels que l'adipate de diisopropyle, l'adipate de dioctyle, les benzoates d'alkyle, le malate de dioctyle.

Afin que les compositions cosmétiques de l'invention soient plus agréables à utiliser (plus douces à l'application, plus nourrissantes, plus émollientes), il est possible d'ajouter une phase grasse dans le milieu de ces compositions.

La phase grasse peut représenter jusqu'à 50% du poids total de la composition.

Cette phase grasse peut comporter une huile ou une cire ou leurs mélanges, et peut comprendre également des acides gras, des alcools gras et des esters d'acides gras. Les huiles peuvent être choisies parmi les huiles animales, végétales, minérales ou de synthèse et notamment parmi l'huile de vaseline, l'huile de paraffine, les isoparaffines, les poly-α-oléfines, les huiles fluorées et perfluorées. De même, les cires peuvent être choisies parmi les cires animales, fossiles, végétales, minérales ou de synthèse connues en soi.

Les compositions de l'invention peuvent contenir des adjuvants habituels dans le domaine cosmétique comme d'autres gélifiants et/ou épaississants classiques; des émulsionnants; des tensio-actifs; des agents hydratants ; des émollients ; des filtres solaires ; des actifs hydrophiles ou lipophiles comme des céramides ; des agents anti-radicaux libres ; des séquestrants ; des antioxydants ; des conservateurs ; des agents alcalinisants ou acidifiants; des parfums; des charges; des matières colorantes; des silicones volatiles ou non, modifiées ou non, des réducteurs. Les quantités de ces différents adjuvants sont celles classiquement utilisées dans les domaines considérés.

Bien entendu, le spécialiste veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous toutes les formes appropriées pour une application topique, notamment sous forme de lotion épaissie, sous forme de gels aqueux ou hydroalcooliques, sous forme de dispersions vésiculaires, sous forme d'émulsions simples ou complexes ((H/E, E/H, H/E/H ou E/H/E) et être de consistance liquide, semi-liquide ou solide telles que des laits, des crèmes, des gels, des gels-crèmes, des pâtes, des sticks et éventuellement être conditionnées en aérosol et se présenter sous la forme de mousses ou de sprays. Ces compositions sont préparées selon les méthodes usuelles.

Les compositions selon l'invention sont de préférence utilisées comme produits capillaires rincés ou non-rincés, notamment pour le lavage, la coloration, le soin, le conditionnement, le défrisage, le maintien de la coiffure ou la mise en forme permanente ou non des cheveux.

Elles peuvent être des produits de coiffage tels que des lotions de mise en plis, des lotions pour le brushing, des compositions de fixation et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray ou une mousse pour la fixation ou le traitement des cheveux.

Les compositions de l'invention peuvent être également des shampooings, des compositions à rincer ou non, à appliquer avant ou après un shampooing, une coloration, une décoloration, une permanente ou un défrisage.

Les compositions de l'invention peuvent être aussi utilisées comme produits de soin ou d'hygiène tels que des crèmes de protection, de traitement ou de soin pour le visage, pour les mains ou pour le corps, des laits corporels de protection ou de soin, des lotions, gels ou mousses pour le soin ou le nettoyage de la peau.

Les compositions de l'invention peuvent aussi être utilisées comme compositions antisolaires.

Les compositions peuvent également consister en des préparations solides constituant des savons ou des pains de nettoyage.

Les compositions de l'invention peuvent être également utilisées comme produits de soin bucco-dentaire tels que des pâtes dentifrices, des bains de bouche.

Les compositions peuvent être des produits pour le maquillage tels que des crèmes pour le visage, des fonds de teint, des mascaras, des eye-liners, des rouges à lèvres, des vernis à ongles.

Un autre objet de l'invention est un procédé de traitement non-thérapeutique cosmétique de la peau, du cuir chevelu, des cheveux, des cils, des sourcils, des ongles ou des muqueuses, caractérisé par le fait qu'on applique sur ces derniers une composition telle que définie ci-dessus, selon la technique d'utilisation habituelle de cette composition, par exemple application de crèmes, de gels, de sérums, de lotions, de laits sur la peau, le cuir chevelu ou les muqueuses.

Les exemples suivants illustrent l'invention sans présenter un caractère limitatif.

### EXEMPLE 1 : Soin capillaire gélifié non-rincé

- Terpolymère acide méthacrylique/méthyl acrylate/diméthyl métaisopropényl benzyl isocyanate d'alcool béhénylique éthoxylé (40 OE) en dispersion aqueuse à 25% 0,5 g MA
- Terpolymère acétate de vinyle/p-tertiobutyl benzoate de vinyle/acide crotonique (65/25/10) 0,5 g MA
- 2-amino 2-méthyl propanol-1 (AMP) pH ajusté à 7.5 qs
- Parfum, conservateur, colorant q.s
- Eau déminéralisée qsp 100 g

On obtient un gel épais, limpide, non pâteux, non gras et s'étalant très bien sur les cheveux. Ce gel donne aux cheveux un toucher doux et une bonne aptitude au démêlage et possède un bon pouvoir fixant.

Si on remplace le terpolymère ci-dessus par la même quantité de polyuréthane "ACRYSOL 44" de ROHM et HAAS, on obtient un gel fluide et trouble.

Si on remplace le terpolymère par le copolymère acide acrylique/acrylate d'alkyle en C₁₀/C₃₀ réticulé "PEMULEN TR1" vendu par GOODRICH, on obtient un gel légèrement pâteux, ayant un pouvoir fixant très médiocre et des propriétés cosmétiques de douceur, toucher et démêlage beaucoup moins bonnes.

### EXEMPLE 2 : Gel solaire haute protection

- 4 tert.butyl 4'-méthoxy dibenzoylméthane ("PARSOL 1789" vendu par la société ROCHE) 2 g
- Acide benzène 1,4-di(3-méthylidène-10-camphosulfonique) en solution aqueuse à 33% 3 g
- 2-cyano-3,3-diphénylacrylate de 2-éthylhexyle ("UVINUL N 539" vendu par la société BASF) 10 g
- Terpolymère acide méthacrylique/méthyl acrylate/diméthyl métaisopropényl benzyl isocyanate d'alcool béhénylique éthoxylé (40 OE) en dispersion aqueuse à 25% 0,45g MA
- Acide polyacrylamidométhyl propane sulfonique réticulé et partiellement neutralisé à 50% par l'ammoniaque, vendu sous la dénomination "HOSTACERIN AMPS" par la société HOECHST 0,8 g
- 2,2,4,4,6,6,8-heptaméthylnonane 9 g
- Glycérol 6 g
- Propylèneglycol 6 g
- Acide éthylène diamine tétra (méthylène phosphonique), sel pentasodique en solution aqueuse à 33% 0,3 g
- Triéthanolamine 0,85g
- Alcool éthylique à 96° dénaturé 4,5 g
- Eau déminéralisée stérilisée qsp 100 g

On obtient un gel onctueux s'étalant bien sur la peau.

## Revendications

1. Composition cosmétique destinée au traitement des matières kératiniques, **caractérisée par le fait qu'**elle comprend, dans un support aqueux cosmétiquement acceptable, au moins un polymère anionique et un terpolymère acrylique, comprenant :
a) 20 à 70% en poids, et de préférence 25 à 55% en poids, d'un acide carboxylique à insaturation α, β-monoéthylénique ;
b) 20 à 65% en poids, et de préférence 30 à 65% en poids, d'un monomère à insaturation monoéthylénique non tensio-actif différent de a) et
c) 0,5 à 60% en poids, et de préférence 10 à 50% en poids, d'un monomère uréthane non-ionique qui est le produit de réaction d'un tensio-actif non-ionique monohydrique avec un monoisocyanate à insaturation monoéthylénique.

2. Composition selon la revendication 1, **caractérisée par le fait que** l'acide carboxylique à insaturation α, β-monoéthylénique a) est choisi parmi l'acide acrylique, l'acide méthacrylique, l'acide itaconique et l'acide maléique.

3. Composition selon la revendication 2, **caractérisée par le fait que** l'acide carboxylique à insaturation α, β-monoéthylénique a) est l'acide méthacrylique.

4. Composition selon l'une quelconque des revendications 1 à 3, **caractérisée par le fait que** le monomère à insaturation monoéthylénique non tensio-actif b) est choisi parmi les acrylates et méthacrylates d'alkyle en C₁-C₄, le styrène, le vinyltoluène, l'acétate de vinyle, l'acrylonitrile et le chlorure de vinylidène.

5. Composition selon la revendication 4, **caractérisée par le fait que** le monomère à insaturation monoéthylénique non tensio-actif est l'acrylate de méthyle ou d'éthyle.

6. Composition selon l'une quelconque des revendications 1 à 5, **caractérisée par le fait que** le tensio-actif non-ionique monohydrique utilisé pour obtenir le monomère uréthane non-ionique c) a pour formule : 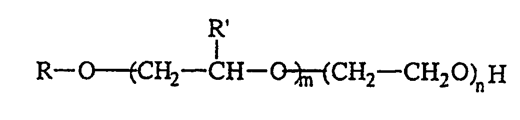 dans laquelle R est un groupe alkyle en C₆-C₃₀ ou aralkyle en C₈-C₃₀, R' est un groupe alkyle en C₁-C₄, n est un nombre moyen allant de 5 à 150 et m est un nombre moyen allant de 0 à 50, à la condition que n soit au moins aussi grand que m et que n + m = 5-150.

7. Composition selon la revendication 6, **caractérisée par le fait que** R est choisi parmi les radicaux dodécyle, alkyle en C₁₈-C₂₆ et alkyl (C₈-C₁₃) phényle, m = 0 et n est un nombre moyen allant d'environ 5 à 150.

8. Composition selon l'une quelconque des revendications 1 à 7, **caractérisée par le fait que** le monoisocyanate à insaturation monoéthylénique utilisé pour former le monomère uréthane non-ionique c) est l'α,α-diméthyl m-isopropényl benzyl isocyanate.

9. Composition selon l'une quelconque des revendications 1 à 8, **caractérisée par le fait que** le terpolymère acrylique est obtenu en dispersion aqueuse à partir d'acide méthacrylique comme composant a), d'acrylate de méthyle comme composant b) et d'un macromonomère uréthane non-ionique de structure suivante : 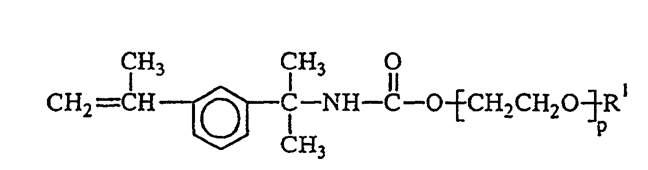 dans laquelle p va de 6 à 150 et R¹ est un radical alkyle en C₁₈-C₂₆, de préférence en C₂₀-C₂₄ linéaire, d'origine végétale, tel que le radical docosyle.

10. Composition selon l'une quelconque des revendications 1 à 9, **caractérisée par le fait que** le terpolymère acrylique est présent dans des concentrations allant de 0,01 à 20% en poids, et de préférence de 0,1 à 10 % en poids, par rapport au poids total de la composition.

11. Composition selon l'une quelconque des revendications 1 à 10, **caractérisée par le fait que** le ou les polymères anioniques sont choisis parmi les polymères comportant des groupements dérivés d'acide carboxylique, sulfonique ou phosphorique.

12. Composition selon la revendication 11, **caractérisée par le fait que** le ou les polymères anioniques sont choisis parmi
- les polymères comportant des groupements carboxyliques dérivant de monomères mono- ou diacides carboxyliques insaturés de formule : 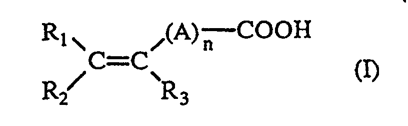 dans laquelle n est un nombre entier de 0 à 10, A désigne un groupe méthylène, éventuellement relié à l'atome de carbone du groupement insaturé ou au groupement méthylène voisin lorsque n est supérieur à 1 par l'intermédiaire d'un hétéroatome tel que l'oxygène ou le soufre, R₁ désigne un atome d'hydrogène, un groupement phényle ou benzyle, R₂ désigne un atome d'hydrogène, un groupement alkyle inférieur ou carboxyle, R₃ désigne un atome d'hydrogène, un groupement alkyle inférieur, un groupement -CH₂ COOH, phényle ou benzyle, et
- les polymères comprenant des motifs vinylsulfonique, styrène sulfonique, naphtalène sulfonique ou acrylamido alkylsulfonique.

13. Composition selon la revendication 12, **caractérisée par le fait que** le ou les polymères anioniques sont choisis parmi:
**A**) les homo- ou copolymères d'acide acrylique ou méthacrylique ou leurs sels, les copolymères d'acide acrylique et d'acrylamide ou leurs sels et les sels de sodium des acides polyhydroxycarboxyliques.
**B**) Les copolymères des acides acrylique ou méthacrylique avec au moins un monomère monoéthylénique choisi parmi l'éthylène, le styrène, les esters vinyliques, les esters d'acide acrylique ou méthacrylique, l'acrylamide ou ses dérivés et la vinylpyrrolidone, éventuellement greffés sur un polyalkylène glycol et éventuellement réticulés.
**C**) Les copolymères dérivés d'acide crotonique comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée comportant au moins 5 atomes de carbone ou un ester vinylique, allylique ou méthallylique d'un acide carboxylique α- ou β-cyclique, ces polymères pouvant éventuellement être greffés sur un polyalkylène glycol et réticulés.
**D**) Les copolymères dérivés d'acides ou d'anhydrides carboxyliques monoinsaturés en C₄-C₈ choisis parmi les copolymères comprenant (i) un ou plusieurs acides ou anhydrides maléique, fumarique ou itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées, et les copolymères comprenant (i) un ou plusieurs anhydrides maléique, citraconique ou itaconique et (ii) un ou plusieurs monomères choisis parmi les esters allyliques ou méthallyliques comportant éventuellement dans leur chaîne un ou plusieurs groupements acrylamide, méthacrylamide, α-oléfine, esters acryliques ou méthacryliques, acides acrylique ou méthacrylique ou vinylpyrrolidone, les fonctions anhydrides étant éventuellement monoestérifiées ou monoamidifiées.
**E**) Les polyacrylamides comportant des groupes carboxylates.

14. Composition selon la revendication 13, **caractérisée par le fait que** le ou les polymères anioniques sont choisis parmi les copolymères d'acide méthacrylique et de méthacrylate de méthyle, les copolymères d'acide méthacrylique et d'acrylate d'éthyle, les terpolymères vinylpyrrolidone/acide acrylique/méthacrylate de lauryle, les terpolymères acide méthacrylique/acrylate d'éthyle/acrylate de tertiobutyle, les terpolymères acide acrylique/acrylate d'éthyle/N-tertiobutylacrylamide, les copolymères acétate de vinyle/acide crotonique, les terpolymères acétate de vinyle/tertiobutylbenzoate de vinyle/acide crotonique, les terpolymères acide crotonique/acétate de vinyle/néododécanoate de vinyle, les terpolymères acétate de vinyle/acide crotonique/polyéthylène glycol et les copolymères méthylvinyléther/anhydride /anhydride maléique monoestérifiés.

15. Composition selon la revendication 14, **caractérisée par le fait que** le ou les polymères anioniques sont choisis parmi les terpolymères acétate de vinyle/tertiobutylbenzoate de vinyle/acide crotonique, les copolymères méthylvinyléther/anhydride maléique monoestérifiés, les copolymères d'acide méthacrylique et de méthacrylate de méthyle, les copolymères d'acide méthacrylique et d'acrylate d'éthyle et les terpolymères vinylpyrrolidone/acide acrylique/méthacrylate de lauryle.

16. Composition selon la revendication 12, **caractérisée par le fait que** le ou les polymères anioniques sont choisis parmi :
- les sels de l'acide polyvinylsulfonique ayant un poids moléculaire moyen en poids compris entre environ 1.000 et 100.000 et leurs copolymères avec les acides acryliques ou méthacryliques ou leurs esters, l'acrylamide et ses dérivés, les éthers vinyliques et la vinylpyrrolidone ;
- les sels de l'acide polystyrène sulfonique ;
- les sels d'acide polyacrylamide alkylsulfonique.

17. Composition selon la revendication 16, **caractérisée par le fait que** le ou les polymères anioniques comprenant des groupements sulfoniques sont choisis parmi l'acide polyacrylamidoéthylpropane sulfonique et l'acide polyacrylamidométhylpropane sulfonique réticulé et partiellement neutralisé à 50% par l'ammoniaque.

18. Composition selon l'une quelconque des revendications 1 à 10, **caractérisée par le fait que** le ou les polymères anioniques sont sous forme d'une dispersion aqueuse de particules insolubles de polymères anioniques choisis parmi les copolymères formés d'un ou plusieurs acrylate d'alkyle, d'un ou plusieurs méthacrylates d'alkyle et d'un ou plusieurs acides carboxyliques éthyléniques ayant de 3 à 5 atomes de carbone, les radicaux alkyle ayant de 1 à 5 atomes de carbone et pouvant être hydroxylés.

19. Composition selon la revendication 18, **caractérisée par le fait que** la dispersion aqueuse de particules insolubles de polymères anioniques comprend un copolymère acrylate d'éthyle/méthacrylate de méthyle/acide méthacrylique/acide acrylique, un copolymère méthacrylate d'hydroxyéthyle/méthacrylate de méthyle/acide méthacrylique/acrylate de butyle ou un copolymère acrylate d'éthyle/acide méthacrylique/acrylate de tert.butyle.

20. Composition selon l'une quelconque des revendications 1 à 19, **caractérisée par le fait que** le ou les polymères anioniques sont présents dans des concentrations allant de 0,01 à 20% en poids, et de préférence de 0,1 à 8% en poids, par rapport au poids total de la composition.

21. Composition selon l'une quelconque des revendications 1 à 20, **caractérisée par le fait qu'**elle présente un pH allant de 3,5 à 11, de préférence de 5,5 à 11, et encore plus préférentiellement de 5,5 à 8,5.

22. Composition selon l'une quelconque des revendications 1 à 21, **caractérisée par le fait que** le milieu aqueux cosmétiquement acceptable est constitué d'eau ou d'eau et au moins un solvant organique choisi dans le groupe constitué par les solvants organiques hydrophiles, lipophiles, amphiphiles ou leurs mélanges.

23. Composition selon l'une quelconque des revendications 1 à 22, **caractérisée par le fait qu'**elle comprend en outre au moins un adjuvant cosmétique usuel choisi parmi les corps gras, les gélifiants et/ou épaississants classiques, les tensio-actifs, les agents hydratants, les émollients, les filtres solaires, les actifs hydrophiles ou lipophiles comme les céramides, les agents anti-radicaux libres, les séquestrants, les antioxydants, les conservateurs, les agents alcalinisants ou acidifiants, les parfums, les charges, les matières colorantes, les silicones et les réducteurs.

24. Composition selon l'une quelconque des revendications 1 à 23, **caractérisée par le fait qu'**elle se présente sous forme d'émulsion, de lotion épaissie, de gel, de dispersion vésiculaire, de pâte, de bâtonnet solide ou est conditionnée en aérosol et se présente sous forme de mousse ou de spray.

25. Composition selon l'une quelconque des revendications 1 à 24, **caractérisée par le fait qu'**elle est utilisée comme produit capillaire rincé ou non-rincé pour le lavage, la teinture, le soin, le conditionnement, le défrisage, le maintien de la coiffure ou la mise en forme permanente ou non des cheveux, ou comme composition anti-solaire.

26. Procédé de traitement non-thérapeutique cosmétique pour le traitement de la peau, du cuir chevelu, des cheveux, des cils, des sourcils, des ongles et des muqueuses, **caractérisé par le fait qu'**on applique sur ces derniers une quantité efficace d'une composition telle que définie selon l'une quelconque des revendications 1 à 25.

## Claims

1. Cosmetic composition intended for the treatment of keratinous material, **characterized in that** it comprises, in a cosmetically acceptable aqueous support, at least one anionic polymer and an acrylic terpolymer comprising:
a) 20 to 70% by weight, and preferably 25 to 55% by weight, of a carboxylic acid containing α, β-monoethylenic unsaturation;
b) 20 to 65% by weight, and preferably 30 to 65% by weight, of a non-surfactant monomer containing monoethylenic unsaturation, which is different from a), and
c) 0.5 to 60% by weight, and preferably 10 to 50% by weight, of a nonionic urethane monomer which is the product of reaction of a monohydric nonionic surfactant with a monoisocyanate containing monoethylenic unsaturation.

2. Composition according to Claim 1, **characterized in that** the carboxylic acid containing α,β-monoethylenic unsaturation a) is chosen from acrylic acid, methacrylic acid, itaconic acid and maleic acid.

3. Composition according to Claim 2, **characterized in that** the carboxylic acid containing α,β-monoethylenic unsaturation a) is methacrylic acid.

4. Composition according to any one of Claims 1 to 3, **characterized in that** the non-surfactant monomer containing monoethylenic unsaturation b) is chosen from C₁-C₄ alkyl acrylates and methacrylates, styrene, vinyltoluene, vinyl acetate, acrylonitrile and vinylidene chloride.

5. Composition according to Claim 4, **characterized in that** the non-surfactant monomer containing monoethylenic unsaturation is methyl or ethyl acrylate.

6. Composition according to any one of Claims 1 to 5, **characterized in that** the monohydric nonionic surfactant used to obtain the nonionic urethane monomer c) has the formula: 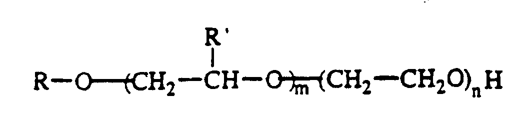 in which R is a C₆-C₃₀ alkyl or C₈-C₃₀ aralkyl group, R' is a C₁-C₄ alkyl group, n is an average number ranging from 5 to 150 and m is an average number ranging from 0 to 50, with the condition that n is at least as large as m and that n+m = 5-150.

7. Composition according to Claim 6, **characterized in that** R is chosen from dodecyl, C₁₈-C₂₆ alkyl and (C₈-C₁₃) alkylphenyl radicals, m = 0 and n is an average number ranging from about 5 to 150.

8. Composition according to any one of Claims 1 to 7, **characterized in that** the monoisocyanate containing monoethylenic unsaturation which is used to form the nonionic urethane monomer c) is α,α-dimethyl-m-isopropenylbenzyl isocyanate.

9. Composition according to any one of Claims 1 to 8, **characterized in that** the acrylic terpolymer is obtained as an aqueous dispersion from methacrylic acid as component a), methyl acrylate as component b) and a nonionic urethane macromonomer of the following structure: 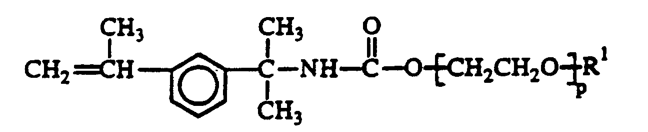 in which p ranges from 6 to 150 and R¹ is a C₁₈-C₂₆, preferably C₂₀-C₂₄, linear alkyl radical of plant origin, such as the docosyl radical.

10. Composition according to any one of Claims 1 to 9, **characterized in that** the acrylic terpolymer is present in concentrations ranging from 0.01 to 20% by weight, and preferably from 0.1 to 10% by weight, relative to the total weight of the composition.

11. Composition according to any one of Claims 1 to 10, **characterized in that** the anionic polymers are chosen from polymers containing groups derived from carboxylic, sulphonic or phosphoric acid.

12. Composition according to Claim 11, **characterized in that** the anionic polymer(s) is (are) chosen from
- polymers containing carboxylic groups derived from unsaturated mono- or dicarboxylic acid monomers of formula: 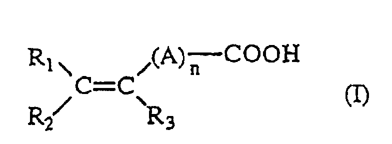 in which n is an integer from 0 to 10, A denotes a methylene group, optionally linked to the carbon atom of the unsaturated group or to the adjacent methylene group when n is greater than 1 via a hetero atom such as oxygen or sulphur, R₁ denotes a hydrogen atom or a phenyl or benzyl group, R₂ denotes a hydrogen atom, a lower alkyl group or a carboxyl group and R₃ denotes a hydrogen atom, a lower alkyl group, a -CH₂COOH group or a phenyl or benzyl group, and
- polymers comprising vinylsulphonic, styrenesulphonic, naphthalenesulphonic or acrylamidoalkylsulphonic units.

13. Composition according to Claim 12, **characterized in that** the anionic polymer(s) is (are) chosen from:
**A**) acrylic or methacrylic acid homo- or copolymers or salts thereof, copolymers of acrylic acid and of acrylamide or salts thereof and the sodium salts of polyhydroxycarboxylic acids.
**B**) copolymers of acrylic or methacrylic acids with at least one monoethylenic monomer chosen from ethylene, styrene, vinyl esters, acrylic or methacrylic acid esters, acrylamide or its derivatives and vinylpyrrolidone, which are optionally grafted onto a polyalkylene glycol and optionally crosslinked.
**C**) Copolymers derived from crotonic acid containing in their chain vinyl acetate or propionate units and optionally other monomers such as allylic or methallylic esters, vinyl ether or vinyl ester of a linear or branched saturated carboxylic acid with a long hydrocarbon-based chain containing at least 5 carbon atoms, or a vinyl, allylic or methallylic ester of an α- or β-cyclic carboxylic acid, it being possible for these polymers to be optionally grafted onto a polyalkylene glycol and crosslinked.
**D**) Copolymers derived from monounsaturated C₄-C₈ carboxylic acids or anhydrides chosen from copolymers comprising (i) one or more maleic, fumaric or itaconic acids or anhydrides and (ii) at least one monomer chosen from vinyl esters, vinyl ethers, vinyl halides, phenylvinyl derivatives and acrylic acid and its esters, the anhydride functions of these copolymers optionally being monoesterified or monoamidated and copolmers comprising (i) one or more maleic, citraconic or itaconic anhydrides and (ii) one or more monomers chosen from allylic or methallylic esters optionally containing in their chain one or more acrylamide, methacrylamide, α-olefin, acrylic or methacrylic ester, acrylic or methacrylic acid or vinylpyrrolidone groups, the anhydride functions optionally being monoesterified or monoamidated.
**E**) Polyacrylamides containing carboxylate groups.

14. Composition according to Claim 13, **characterized in that** the anionic polymer(s) is (are) chosen. from copolymers of methacrylic acid and of methyl methacrylate, copolymers of methacrylic acid and of ethyl acrylate, vinylpyrrolidone/acrylic acid/lauryl methacrylate terpolymers, methacrylic acid/ethyl acrylate/tert-butyl acrylate terpolymers, acrylic acid/ethyl acrylate/N-tert-butylacrylamide terpolymers, vinyl acetate/crotonic acid copolymers, vinyl acetate/vinyl tert-butylbenzoate/crotonic acid terpolymers, crotonic acid/vinyl acetate/vinyl neododecanoate terpolymers, vinyl acetate/crotonic acid/polyethylene glycol terpolymers and monoesterified methyl vinyl ether/maleic anhydride copolymers.

15. Composition according to Claim 14, **characterized in that** the anionic polymer(s) is (are) chosen from vinyl acetate/vinyl tert-butylbenzoate/crotonic acid terpolymers, monoesterified methyl vinyl ether/maleic anhydride copolymers, copolymers of methacrylic acid and of methyl methacrylate, copolymers of methacrylic acid and of ethyl acrylate and vinylpyrrolidone/acrylic acid/lauryl methacrylate terpolymers.

16. Composition according to Claim 12, **characterized in that** the anionic polymer(s) is (are) chosen from:
- polyvinylsulphonic acid salts having a weight-average molecular weight of between about 1000 and 100,000 and copolymers thereof with acrylic or methacrylic acids or their esters, acrylamide and its derivatives, vinyl ethers and vinylpyrrolidone;
- polystyrenesulphonic acid salts;
- polyacrylamidealkylsulphonic acid salts.

17. Composition according to Claim 16, **characterized in that** the anionic polymer(s) comprising sulphonic groups is (are) chosen from polyacrylamidoethylpropanesulphonic acid and polyacrylamidomethylpropanesulphonic acid crosslinked and partially neutralized to 50% with aqueous ammonia.

18. Composition according to any one of Claims 1 to 10, **characterized in that** the anionic polymer(s) is (are) in the form of an aqueous dispersion of insoluble particles of anionic polymers chosen from copolymers formed of one or more alkyl acrylates, of one or more alkyl methacrylates and of one or more ethylenic carboxylic acids having from 3 to 5 carbon atoms, the alkyl radicals having from 1 to 5 carbon atoms and possibly being hydroxylated.

19. Composition according to Claim 18, **characterized in that** the aqueous dispersion of insoluble particles of anionic polymers comprises an ethyl acrylate/methyl methacrylate/methacrylic acid/acrylic acid copolymer, a hydroxyethyl methacrylate/methyl methacrylate/methacrylic acid/butyl acrylate copolymer or an ethyl acrylate/methacrylic acid/tert-butyl acrylate copolymer.

20. Composition according to any one of Claims 1 to 19, **characterized in that** the anionic polymer(s) is (are) present in concentrations ranging from 0.01 to 20% by weight, and preferably from 0.1 to 8% by weight, relative to the total weight of the composition.

21. Composition according to any one of Claims 1 to 20, **characterized in that** it has a pH ranging from 3.5 to 11, preferably from 5.5 to 11 and even more preferably from 5.5 to 8.5.

22. Composition according to any one of Claims 1 to 21, **characterized in that** the cosmetically acceptable aqueous medium consists of water or of water and at least one organic solvent chosen from the group consisting of hydrophilic, lipophilic and amphiphilic organic solvents or mixtures thereof.

23. Composition according to any one of Claims 1 to 22, **characterized in that** it also comprises at least one common cosmetic adjuvant chosen from fatty substances, standard gelling agents and/or thickeners, surfactants, moisturizers, emollients, sunscreens, hydrophilic or lipophilic active agents such as ceramides, anti-free-radical agents, sequestering agents, antioxidants, preserving agents, acidifying or basifying agents, fragrances, fillers, dyestuffs, silicones and reducing agents.

24. Composition according to any one of Claims 1 to 23, **characterized in that** it is in the form of an emulsion, a thickened lotion, a gel, a vesicle dispersion, a paste or a solid stick or is packaged as an aerosol and is in the form of a mousse or a spray.

25. Composition according to any one of Claims 1 to 24, **characterized in that** it is used as a rinse-out or leave-in hair product to wash, dye, care for, condition or straighten the hair, to maintain the hairstyle or to permanently or temporarily reshape the hair, or as an antisun composition.

26. Cosmetic, non-therapeutic treatment process for treating the skin, the scalp, the hair, the eyelashes, the eyebrows, the nails and mucous membranes, **characterized in that** an effective amount of a composition as defined according to any one of Claims 1 to 25 is applied to these materials.

## Patentansprüche

1. Kosmetische Zusammensetzung, die für die Behandlung von Keratinmaterialien vorgesehen ist, **dadurch gekennzeichnet, daß** sie in einem kosmetisch akzeptablen Träger mindestens ein anionisches Polymer und ein Acrylterpolymer enthält, das enthält:
a) 20 bis 70 Gew.-% und vorzugsweise 25 bis 55 Gew.-% einer α,β-monoethylenisch ungesättigten Carbonsäure,
b) 20 bis 65 Gew.-% und vorzugsweise 30 bis 65 Gew.-% eines nicht tensidisch wirkenden, von a) verschiedenen monoethylenisch ungesättigten Monomers und
c) 0,5 bis 60 Gew.-% und vorzugsweise 10 bis 50 Gew.-% eines nicht-ionischen Urethanmonomers, das das Produkt der Umsetzung eines nicht-ionischen, ein reaktives Wasserstoffatom aufweisenden grenzflächenaktiven Stoffes mit einem monoethylenisch ungesättigten Monoisocyanat ist.

2. Zusammensetzung nach Anspruch 1, **dadurch gekennzeichnet, daß** die α,β-monoethylenisch ungesättigte Carbonsäure a) unter Acrylsäure, Methacrylsäure, Itaconsäure und Maleinsäure ausgewählt ist.

3. Zusammensetzung nach Anspruch 2, **dadurch gekennzeichnet, daß** die α,β-monoethylenisch ungesättigte Carbonsäure a) die Methacrylsäure ist.

4. Zusammensetzung nach einem der Ansprüche 1 bis 3, **dadurch gekennzeichnet, daß** das nicht tensidisch wirkende, monoethylenisch ungesättigte Monomer unter den C₁-C₄-Alkylacrylaten und C₁-C₄-Alkylmethacrylaten, Styrol, Vinyltoluol, Vinylacetat, Acrylnitril und Vinylidenchlorid ausgewählt ist.

5. Zusammensetzung nach Anspruch 4, **dadurch gekennzeichnet, daß** es sich bei dem nicht tensidisch wirkenden, monoethylenisch ungesättigten Monomer um Methylacrylat oder Ethylacrylat handelt.

6. Zusammensetzung nach einem der Ansprüche 1 bis 5, **dadurch gekennzeichnet, daß** der nicht-ionische, ein reaktives Wasserstoffatom aufweisende grenzflächenaktive Stoff, der verwendet wird, um das nicht-ionische Urethanmonomer c) zu erhalten, die folgende Formel 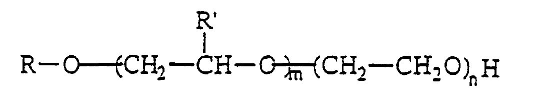 hat, in der R eine C₆-C₃₀-Alkylgruppe oder C₈-C₃₀-Aralkylgruppe ist, R' eine C₁-C₄-Alkylgruppe bedeutet, n ein Mittelwert ist, der im Bereich von 5 bis 150 liegt und m ein Mittelwert ist, der im Bereich von 0 bis 50 liegt, mit der Maßgabe, daß n mindestens genauso groß wie m ist und daß n + m = 5-150.

7. Zusammensetzung nach Anspruch 6, **dadurch gekennzeichnet, daß** R unter Dodecyl, C₁₈-C₂₆-Alkyl und Phenyl-(C₈-C₁₃)-alkyl ausgewählt ist, m = 0 und n ein Mittelwert im Bereich von etwa 5 bis 150 ist.

8. Zusammensetzung nach einem der Ansprüche 1 bis 7, **dadurch gekennzeichnet, daß** das monoethylenisch ungesättigte Monoisocyanat, das für die Herstellung des nicht-ionischen Urethanmonomers c) verwendet wird, das α,α-Dimethyl-m-isopropenylbenzylisocyanat ist.

9. Zusammensetzung nach einem der Ansprüche 1 bis 8, **dadurch gekennzeichnet, daß** das Acrylterpolymer in wäßriger Dispersion aus Methacrylsäure als Bestandteil a), Methylacrylat als Bestandteil b) und einem nicht-ionischen Urethan-Makromonomer der folgenden Struktur 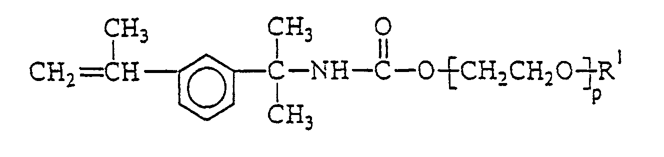 hergestellt wird, in der p im Bereich von 6 bis 150 liegt und R¹ ein C₁₈-C₂₆-Alkylrest, vorzugsweise ein geradkettiger C₂₀-C₂₄-Alkylrest, pflanzlicher Herkunft, wie Docosyl, ist.

10. Zusammensetzung nach einem der Ansprüche 1 bis 9, **dadurch gekennzeichnet, daß** das Acrylterpolymer in einer Konzentration von 0,01 bis 20 Gew.-%, und vorzugsweise 0,1 bis 10 Gew.-%, bezogen auf das Gesamtgewicht der Zusammensetzung, enthalten ist.

11. Zusammensetzung nach einem der Ansprüche 1 bis 10, **dadurch gekennzeichnet, daß** das oder die anionischen Polymere unter den Polymeren ausgewählt sind, die Gruppen aufweisen, die von Carbonsäure, Sulfonsäure oder Phosphorsäure stammen.

12. Zusammensetzung nach Anspruch 11, **dadurch gekennzeichnet, daß** das oder die anionischen Polymere ausgewählt sind unter
- den Polymeren, die Carbonsäuregruppen aufweisen, die von ungesättigten Mono- oder Dicarbonsäure-Monomeren der Formel 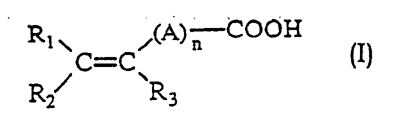 stammen, in der bedeuten: n eine ganze Zahl von 0 bis 10, A eine Methylengruppe, die gegebenenfalls mit dem Kohlenstoffatom der ungesättigten Gruppe oder der benachbarten Methylengruppe, wenn n größer als 1 ist, über ein Heteroatom, wie Sauerstoff oder Schwefel verbunden ist, Ri ein Wasserstoffatom, eine Phenyl- oder Benzylgruppe, R₂ ein Wasserstoffatom, niederes Alkyl oder eine Carboxygruppe, R₃ ein Wasserstoffatom, niederes Alkyl, eine Gruppe -CH₂COOH, Phenyl oder Benzyl, und
- den Polymeren, die Vinylsulfon-, Styrolsulfon-, Naphthalinsulfon- oder Acrylamidoalkylsulfon-Einheiten enthalten.

13. Zusammensetzung nach Anspruch 12, **dadurch gekennzeichnet, daß** das oder die anionischen Polymere ausgewählt sind unter:
A) den Homo- oder Copolymeren der Acrylsäure oder Methacrylsäure oder ihren Salzen, den Copolymeren aus Acrylsäure und Acrylamid oder ihren Salzen und den Natriumsalzen der Polyhydroxycarbonsäuren,
B) den Copolymeren aus Acrylsäure oder Methacrylsäure und mindestens einem monoethylenischen Monomer, das unter Ethylen, Styrol, den Vinylestern, den Acrylsäurestern oder Methacrylsäureestern, Acrylamid oder seinen Derivaten und Vinylpyrrolidon ausgewählt ist, die gegebenenfalls auf ein Polyalkylenglykol gepfropft und gegebenenfalls vernetzt sind.
C) den Copolymeren, die von Crotonsäure abstammen, die in ihrer Kette Vinylacetat- oder Vinylpropionat-Einheiten und gegebenenfalls andere Monomere, wie Allylester oder Methallylester, Vinylether oder Vinylester einer gesättigten, geradkettigen oder verzweigten Carbonsäure mit langer Kohlenwasserstoffkette, die mindestens 5 Kohlenstoffatome aufweist, oder einen Vinylester, Allylester oder Methallylester einer α- oder β-cyclischen Carbonsäure enthalten, wobei diese Polymere gegebenenfalls auf einen Polyalkylenglykol gepfropft und vernetzt sein können;
D) den Copolymeren, die von einfach ungesättigten C₄-C₈-Carbonsäuren oder C₄-C₈-Carbonsäureanhydriden abstammen, die ausgewählt sind unter den Copolymeren, die enthalten: (i) Maleinsäure und/oder Fumarsäure und/oder Itaconsäure und/oder ein oder mehrere Anhydride dieser Säuren und (ii) mindestens ein Monomer, das unter den Vinylestern, Vinylethern, Vinylhalogeniden, Phenylvinyl-Derivaten, der Acrylsäure und ihren Estern ausgewählt ist, wobei die Anhydridgruppen dieser Copolymere gegebenenfalls einfach verestert oder einfach amidiert sind, und den Copolymeren, die enthalten: (i) Maleinsäuresäure und/oder Fumarsäure und/oder Itaconsäure und/oder ein oder mehrere Anhydride dieser Säuren und (ii) ein oder mehrere Monomere, die ausgewählt sind unter den Allylestern oder Methallylestern, die gegebenenfalls in ihrer Kette ein oder mehrere Acrylamid-, Methacrylamid-, α-Olefin-, Acrylester- oder Methacrylester-, Acrylsäure- oder Methacrylsäure- oder Vinylpyrrolidon-Gruppen enthalten, wobei die Anhydrid-Gruppen gegebenenfalls einfach verestert oder einfach amidiert sind,
E) den Polyarylamiden, die Carboxylat-Gruppen enthalten.

14. Zusammensetzung nach Anspruch 13, **dadurch gekennzeichnet, daß** das oder die anionischen Polymere unter den Methacrylsäure/Methylmethacrylat-Copolymeren, den Methacrylsäure/Ethylacrylat-Copolymeren, den Vinylpyrrolidon/Acrylsäure/Laurylmethacrylat-Terpolymeren, den Methacrylsäure/Ethylacrylat/*tert*.-Butylacrylat-Terpolymeren, den Acrylsäure/ Ethylacrylat/ N-*tert*. -Butylacrylamid-Terpolymeren, den Vinylacetat/ Crotonsäure-Copolymeren, den Vinylacetat/Crotonsäure-Copolymeren, den Vinylacetat/-Vinyl-*tert*.-Butylbenzoat/Crotonsäure-Terpolymeren, den Crotonsäure/Vinylacetat/Vinylneododecanoat-Terpolymeren, den Vinylacetat/Crotonsäure/Polyethylenglykol-Terpolymeren und den Copolymeren aus Methylvinylether und einfach verestertem Maleinsäureanhydrid ausgewählt sind.

15. Zusammensetzung nach Anspruch 14, **dadurch gekennzeichnet, daß** das oder die anionischen Polymere unter den Vinylacetat/Vinyl-*tert*.-Butylbenzoat/Crotonsäure-Terpolymeren, den Copolymeren aus Methylvinylether und einfach verestertem Maleinsäureanhydrid, den Methacrylsäure/Methylmethacrylat-Copolymeren, den Methacrylsäure/Ethylacrylat-Copolymeren und den Vinylpyrrolidon/Acrylsäure/Laurylmethacrylat-Terpolymeren ausgewählt sind.

16. Zusammensetzung nach Anspruch 12, **dadurch gekennzeichnet, daß** das oder die anionischen Polymere ausgewählt sind unter:
- den Salzen der Polyvinylsulfonsäure, die ein Gewichtsmittel des Molekulargewichts von etwa 1000 bis 100000 aufweist, und ihrer Copolymere mit Acrylsäure oder Methacrylsäure oder deren Estern, Acrylamid und seinen Derivaten, Vinylethern und Vinylpyrrolidon;
- den Salzen der Polystyrolsulfonsäure;
- den Salzen der Polyacrylamidalkylsulfonsäure.

17. Zusammensetzung nach Anspruch 16, **dadurch gekennzeichnet, daß** das oder die anionischen Polymere, die Sulfongruppen enthalten, unter der Polyacrylamidoethylpropansulfonsäure und der Polyacrylamidomethylpropansulfonsäure, die vernetzt und teilweise zu 50% mit Ammoniak neutralisiert ist, ausgewählt sind.

18. Zusammensetzung nach einem der Ansprüche 1 bis 10, **dadurch gekennzeichnet, daß** das oder die anionischen Polymere in Form einer wäßrigen Dispersion unlöslicher Partikel aus anionischen Polymeren vorliegen, die unter den Copolymeren ausgewählt sind, die aus einem oder mehreren Alkylacrylaten, einem oder mehreren Alkylmethacrylaten und einer oder mehreren ethylenischen Carbonsäuren, wobei die Alkylreste 1 bis 5 Kohlenstoffatome aufweisen und hydroxyliert sein können, gebildet sind.

19. Zusammensetzung nach Anspruch 18, **dadurch gekennzeichnet, daß** die wäßrige Dispersion unlöslicher Partikel aus anionischen Polymeren ein Ethylacrylat/Methylmethacrylat/Methacrylsäure/Acrylsäure-Copolymer, ein Hydroxyethylmethacrylat/Methylmethacrylat/Methacrylsäure/Butylacrylat-Copolymer oder ein Ethylacrylat/Methacrylsäure/*tert*.-Butylacrylat-Copolymer enthält.

20. Zusammensetzung nach einem der Ansprüche 1 bis 19, **dadurch gekennzeichnet, daß** das oder die anionischen Polymere in einer Konzentration im Bereich von 0,01 bis 20 Gew.-%, vorzugsweise 0,1 bis 8 Gew.-%, bezogen auf das Gesamtgewicht der Zusammensetzung, enthalten sind.

21. Zusammensetzung nach einem der Ansprüche 1 bis 20, **dadurch gekennzeichnet, daß** sie einen pH-Wert im Bereich von 3,5 bis 11, vorzugsweise 5,5 bis 11 und noch bevorzugter 5,5 bis 8,5 aufweist.

22. Zusammensetzung nach einem der Ansprüche 1 bis 21, **dadurch gekennzeichnet, daß** das kosmetisch akzeptable wäßrige Medium aus Wasser oder Wasser und mindestens einem organischen Lösemittel, das unter den hydrophilen, lipophilen, amphiphilen organischen Lösemitteln oder ihren Gemischen ausgewählt wird, besteht.

23. Zusammensetzung nach einem der Ansprüche 1 bis 22, **dadurch gekennzeichnet**, daß'sie außerdem mindestens einen üblichen kosmetischen Hilfsstoff enthält, der unter den Fettsubstanzen, den herkömmlichen Gelbildnern und/oder Verdickungsmitteln, den grenzflächenaktiven Stoffen, den Hydratisierungsmitteln, den Emollientien, den Sonnenschutzfiltern, den hydrophilen oder lipophilen Wirkstoffen, wie den Ceramiden, den Mitteln gegen freie Radikale, den Maskierungsmitteln, den Antioxidantien, den Konservierungsmitteln, den alkalisch machenden Mitteln oder Säuerungsmitteln, den Parfüms, den Füllstoffen, den Farbmitteln, den Siliconen und den Reduktionsmitteln ausgewählt wird.

24. Zusammensetzung nach einem der Ansprüche 1 bis 23, **dadurch gekennzeichnet, daß** sie in Form einer Emulsion, einer verdickten Lotion, eines Gels, einer Vesikeldispersion, einer Paste oder Masse, eines festen Stifts vorliegt oder als Aerosol verpackt ist und in Form eines Schaumes oder Sprays vorliegt.

25. Zusammensetzung nach einem der Ansprüche1 bis 24, **dadurch gekennzeichnet, daß** sie als Haarbehandlungsprodukt, das ausgespült oder nicht ausgespült wird, zum Waschen, Färben, Pflegen, Konditionieren, zur Frisurenentfernung, zur Festigung der Frisur, zur Verformung der Haare, die dauerhaft oder nicht dauerhaft ist, oder als Sonnenschutzzusammensetzung verwendet wird.

26. Nicht-therapeutisches kosmetisches Behandlungsverfahren zur Behandlung der Haut, der Kopfhaut, der Haare, der Wimpern, der Augenbrauen, der Nägel und der Schleimhäute, **dadurch gekennzeichnet, daß** auf letztere eine wirksame Menge einer wie in einem der Ansprüche 1 bis 25 definierten Zusammensetzung aufgetragen wird.
